# FASCICULE DE BREVET EUROPEEN

(11) **EP 1 190 701 B1**
(45) Date de publication et mention de la délivrance du brevet: **09.02.2005**
(21) Numéro de dépôt: 01402397.2
(22) Date de dépôt: 19.09.2001
(51) Int. Cl.: A61K 7/11

(54) **Composition conditionnée dans un dispositif aérosol, comprenant des nanoparticules d'alumine**
Zusammensetzung in einem Aerosolbehälter, die Aluminiumoxyd-Nanoteilchen enthält
Composition, packaged in an aerosol device, comprising alumina nanoparticles

(30) Priorité: 20.09.2000 FR 0011991
(43) Date de publication de la demande: 27.03.2002
(73) Titulaire: L'OREAL, 75008 Paris (FR)
(72) Inventeur: Nocerino, Cécile, 75006 Paris (FR); Giroud, Franck, 92110 Clichy (FR); Sturla, Jean-Michel, 92100 Boulogne (FR)
(74) Mandataire: Casalonga, Axel

(56) Documents cités:
- PATENT ABSTRACTS OF JAPAN vol. 7, no. 205 (C-185), 9 septembre 1983 (1983-09-09) & JP 58 103301 A (TOUYOU AEROSOL KOGYO KK), 20 juin 1983 (1983-06-20)
- PATENT ABSTRACTS OF JAPAN vol. 2000, no. 11, 3 janvier 2001 (2001-01-03) & JP 2000 212051 A (OSAKA SHIP BUILDING CO), 2 août 2000 (2000-08-02)

## Description

La présente invention est relative à une composition conditionnée dans un dispositif aérosol comprenant un agent propulseur et une phase liquide qui contient, dans un milieu cosmétiquement acceptable, des nanoparticules d'alumine, à un procédé de traitement cosmétique des cheveux et à une utilisation en tant que produit de coiffage.

Des produits tels que, par exemple, des laques, des mousses et des gels, sont bien connus dans la technique pour coiffer les cheveux en les fixant entre eux.

Cependant, un simple passage des doigts, d'un peigne ou d'une brosse entraîne une cassure des soudures créées lors de l'application de ces produits classiques. Ils ne permettent donc pas un recoiffage des cheveux.

Le brevet US 3 819 827 de WELLA décrit en particulier des produits pour la mise en plis des cheveux comprenant de 0,2 à 6 % en poids de particules d'oxyde d'aluminium présentant une taille de particule d'environ 30 mµ.

La Demanderesse a trouvé de manière surprenante et inattendue que l'utilisation en aérosol de nanoparticules contenant au moins 10 % en poids d'alumine et présentant une taille primaire moyenne en nombre comprise entre 2 et 200 nm, dans un milieu cosmétiquement acceptable, permettait de donner du volume à la chevelure et d'obtenir un coiffage sans fixer et surcharger les cheveux. Les cheveux peuvent alors être coiffés et recoiffés à volonté tout en conservant un aspect naturel.

Par milieu cosmétiquement acceptable, on entend un milieu compatible avec les cheveux, mais aussi d'odeur, d'aspect et de toucher agréables.

L'invention a donc pour objet une composition conditionnée dans un dispositif aérosol comprenant un agent propulseur particulier et une phase liquide qui contient, dans un milieu cosmétiquement acceptable, des nanoparticules contenant au moins 10 % en poids d'alumine et présentant une taille primaire moyenne en nombre comprise entre 2 et 200 nm.

Un autre objet de la présente invention consiste en un procédé de traitement cosmétique des cheveux mettant en oeuvre la composition selon l'invention.

L'invention a encore pour objet une utilisation de la composition selon l'invention comme produit de coiffage.

D'autres objets, caractéristiques, aspects et avantages de l'invention apparaîtront encore plus clairement à la lecture de la description et des divers exemples qui suivent.

Selon l'invention, la composition cosmétique conditionnée dans un dispositif aérosol comprend un agent propulseur choisi parmi le diméthyléther, les alcanes en C₃₋₅, le 1,1-difluoroéthane, les mélanges de diméthyléther et d'alcanes en C₃₋₅, les mélanges de 1,1-difluoroéthane et de diméthyléther et/ou d'alcanes en C_{3-5,} et une phase liquide qui contient, dans un milieu cosmétiquement acceptable, des nanoparticules contenant au moins 10 % en poids d'alumine et présentant une taille primaire moyenne en nombre comprise entre 2 et 200 nm, et de préférence entre 5 et 50 nm.

Les particules selon l'invention peuvent, par exemple, avoir une forme quelconque, par exemple la forme de sphères, de paillettes, d'aiguilles, de plaquettes ou des formes totalement aléatoires.

Au sens de la présente invention, on entend par "taille primaire de particule", la dimension maximale qu'il est possible de mesurer entre deux points diamétralement opposés d'une particule individuelle. La taille peut être déterminée par microscopie électronique à transmission ou à partir de la mesure de la surface spécifique par la méthode BET.

Conformément à la présente invention, les particules solides contenant de l'alumine forment une masse dans laquelle l'alumine ne sert pas d'agent d'enrobage à une (ou d'autres) charge(s).

Dans le cas où les nanoparticules sont formées par de l'alumine et d'autres charges, l'alumine se trouve à l'état libre et ne forme pas de liaisons chimiques avec les autres charges. Il s'agit alors d'un alliage entre l'alumine et d'autres charges, notamment avec des oxydes de métaux ou de métalloïdes, en particulier obtenu par fusion thermique de ces différents constituants.

Lorsque les nanoparticules contenant au moins 10 % en poids d'alumine comprennent en outre un oxyde de métal ou de métalloïde autre que l'alumine, celui-ci est notamment choisi parmi l'oxyde de silicium ou de bore.

De préférence, les nanoparticules contiennent au moins 50 % en poids d'alumine, mieux encore au moins 70 % en poids, et les nanoparticules constituées à plus de 90 % en poids d'alumine sont particulièrement préférées selon la présente invention.

L'alumine convenant dans les compositions de la présente invention est de préférence une alumine éventuellement hydratée, telle que, par exemple, la boehmite.

Les nanoparticules contenant de l'alumine selon l'invention sont notamment utilisées en une quantité comprise entre 0,01 % et 30 % en poids, et de préférence entre 0,05 % et 5 % en poids par rapport au poids total de la composition.

La phase liquide peut également contenir d'autres types de nanoparticules, par exemple d'oxyde de titane ou de zinc.

Le milieu cosmétiquement acceptable comprend de l'eau et/ou un solvant cosmétiquement acceptable notamment choisi parmi les alcools inférieurs en C₁-C₄, comme l'éthanol, l'isopropanol, le tertio-butanol et le n-butanol ; les polyols comme le propylèneglycol ; les éthers de polyols ; l'acétone ; et leurs mélanges. Le solvant particulièrement préféré dans l'invention est l'éthanol.

Ce milieu cosmétiquement acceptable comprend de préférence une quantité d'eau inférieure à 20 % en poids de la composition.

La phase liquide de la composition selon l'invention peut comprendre en outre des additifs cosmétiques usuels choisis parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les tensioactifs, les polymères fixants ou non, les silicones volatiles ou non, les huiles végétales, animales, minérales ou de synthèse, les protéines et les vitamines, et leurs mélanges.

A titre d'additif particulièrement préféré, on peut citer, par exemple, les homopolymères ou les copolymères de vinyllactame bien connus dans la technique.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

L'agent propulseur est notamment présent en une quantité comprise entre 2 et 90 % en poids, de préférence entre 40 et 90 %, mieux encore entre 40 et 80 % en poids par rapport au poids total de la composition.

De préférence, le dispositif aérosol selon l'invention comprend comme agent propulseur du diméthyléther seul ou en mélange.

Les compositions conformes à l'invention sont conditionnées dans un dispositif aérosol usuel en cosmétique. Les compositions pulvérisées peuvent se présenter sous la forme d'un spray ou d'une mousse.

Les compositions conformes à l'invention, pulvérisées à partir du dispositif aérosol, peuvent être utilisées en application rincée ou non, comme compositions de fixation et/ou de maintien des cheveux, compositions de soin de cheveux, shampooings, compositions de conditionnement des cheveux, telles que des compositions destinées à apporter de la douceur aux cheveux, ou encore des compositions de maquillage des cheveux.

La présente invention concerne également un procédé de traitement cosmétique des cheveux qui consiste à appliquer une quantité efficace d'une composition telle que décrite ci-dessus, sur les cheveux et à rincer ou non après un éventuel temps de pose.

Selon un mode de réalisation préféré de l'invention, la composition pulvérisée à partir du dispositif aérosol peut être utilisée comme produit de coiffage non rincé.

L'exemple suivant illustre la présente invention.

### Exemple

On a préparé un produit de coiffage à partir des ingrédients suivants, les pourcentages étant exprimés en poids :

| | |
|---|---|
| Nanopigment d'oxyde d'aluminium⁽¹⁾ | 0,3 % |
| Eau | 2,7 % |
| Alcool éthylique | 32 % |
| Diméthyléther | 65 % |

| | |
|---|---|
| ⁽¹⁾ présentant une taille de particule primaire moyenne en nombre de 13 nm, vendu sous la dénomination ALUMINIUMOXID C par la société DEGUSSA-HULS. | |

On mélange les ingrédients dans un bidon muni d'une valve et d'un diffuseur. On pulvérise cette composition sur les cheveux et on laisse sécher quelques secondes.

Les cheveux présentent beaucoup de volume. Ils sont lisses, faciles à coiffer et à recoiffer.

## Revendications

1. Composition conditionnée dans un dispositif aérosol, comprenant une phase liquide qui comporte, dans un milieu cosmétiquement acceptable, des nanoparticules contenant au moins 10 % en poids d'alumine et présentant une taille primaire moyenne en nombre comprise entre 2 et 200 nm, et un agent propulseur choisi parmi le diméthyléther, les alcanes en C₃₋₅, le 1,1-difluoroéthane, les mélanges de diméthyléther et d'alcanes en C₃₋₅, les mélanges de 1,1-difluoroéthane et de diméthyléther et/ou d'alcanes en C₃₋₅.

2. Composition selon la revendication 1, **caractérisée en ce que** la taille primaire moyenne en nombre des nanoparticules est comprise entre 5 et 50 nm.

3. Composition selon la revendication 1 ou 2, **caractérisée en ce que** les nanoparticules contenant au moins 10 % en poids d'alumine comprennent en outre un oxyde de métal ou de métalloïde autre que l'alumine.

4. Composition selon la revendication 3, **caractérisée en ce que** l'oxyde de métal ou de métalloïde est choisi parmi l'oxyde de silicium ou de bore.

5. Composition selon l'une quelconque des revendications précédentes, **caractérisée en ce que** les nanoparticules contiennent au moins 50 % en poids en poids d'alumine.

6. Composition selon l'une quelconque des revendications précédentes, **caractérisée en ce que** les nanoparticules sont constituées à plus de 90 % en poids d'alumine.

7. Composition selon l'une quelconque des revendications précédentes, **caractérisée en ce que** l'alumine est une alumine éventuellement hydratée.

8. Composition selon la revendication 7, **caractérisée en ce que** l'alumine est la boehmite.

9. Composition selon l'une quelconque des revendications précédentes, **caractérisée en ce que** la quantité des nanoparticules est comprise entre 0,01 et 30 % en poids, de préférence entre 0,05 et 5 % en poids par rapport au poids de la composition.

10. Composition selon l'une quelconque des revendications précédentes, **caractérisée en ce que** l'agent propulseur est constitué par du diméthyléther seul ou en mélange.

11. Composition selon l'une quelconque des revendications précédentes, **caractérisée en ce que** l'agent propulseur est contenu en une quantité comprise entre 2 et 90 % en poids, de préférence entre 40 et 80 % en poids par rapport au poids total de la composition.

12. Composition selon l'une quelconque des revendications précédentes, **caractérisée en ce que** le milieu cosmétiquement acceptable comprend de l'eau et/ou un solvant cosmétiquement acceptable.

13. Composition selon la revendication 12, **caractérisée en ce que** le solvant est choisi parmi les alcools inférieurs en C₁₋₄, les polyols, les éthers de polyols, l'acétone et leurs mélanges.

14. Composition selon la revendication 13, **caractérisée en ce que** le solvant est l'éthanol.

15. Composition selon la revendication 12, **caractérisée en ce que** l'eau représente moins de 20 % en poids de la composition.

16. Composition selon l'une quelconque des revendications précédentes, **caractérisée en ce que** la phase liquide comprend en outre des additifs cosmétiques usuels choisis parmi les agents adhésifs, les agents réducteurs comme les thiols, les corps gras, les agents épaississants, les adoucissants, les agents anti-mousse, les filtres, les agents antiperspirants, les agents acidifiants, les agents alcalinisants, les colorants, les pigments, les parfums, les conservateurs, les tensioactifs, , les polymères fixants ou non, les silicones volatiles ou non, les huiles végétales, animales ou minérales, les protéines et les vitamines, et leurs mélanges.

17. Composition selon la revendication 16, **caractérisée en ce qu'**elle contient un homopolymère ou un copolymère de vinyllactame.

18. Procédé de traitement cosmétique des cheveux, **caractérisé en ce que** l'on applique la composition selon l'une quelconque des revendications précédentes sur les cheveux et que l'on rince ou non après un éventuel temps de pose.

19. Utilisation de la composition selon l'une quelconque des revendications 1 à 17, pulvérisée à partir du dispositif aérosol, comme produit de coiffage de préférence non rincé.

## Patentansprüche

1. Zusammensetzung, die in einer Aerosolvorrichtung konfektioniert ist, welche ein flüssige Phase, die in einem kosmetisch akzeptablen Medium Nanopartikel aufweist, die mindestens 10 Gew.-% Aluminiumoxid enthalten und eine zahlenmittlere Größe der Primärteilchen im Bereich von 2 bis 200 nm aufweisen, und ein Treibmittel enthält, das unter Dimethylether, C₃₋₅-Alkanen, 1,1-Difluorethan, Gemischen von Dimethylether und C₃₋₅-Alkanen und Gemischen von 1,1-Difluorethan und Dimethylether und/oder C₃₋₅-Alkanen ausgewählt ist.

2. Zusammensetzung nach Anspruch 1, **dadurch gekennzeichnet, dass** die zahlenmittlere Größe der Primärteilchen der Nanopartikel im Bereich von 5 bis 50 nm liegt.

3. Zusammensetzung nach Anspruch 1 oder 2, **dadurch gekennzeichnet, dass** die Nanopartikel, die mindestens 10 Gew.-% Aluminiumoxid enthalten, ferner ein Metalloxid oder Halbmetalloxid enthalten, das von Aluminiumoxid verschieden ist.

4. Zusammensetzung nach Anspruch 3, **dadurch gekennzeichnet, dass** das Metalloxid oder Halbmetalloxid unter Siliciumoxid oder Boroxid ausgewählt ist.

5. Zusammensetzung nach einem der vorhergehenden Ansprüche, **dadurch gekennzeichnet, dass** die Nanopartikel mindestens 50 Gew.-% Aluminiumoxid enthalten.

6. Zusammensetzung nach einem der vorhergehenden Ansprüche, **dadurch gekennzeichnet, dass** die Nanopartikel zu mehr als 90 Gew.-% aus Aluminiumoxid bestehen.

7. Zusammensetzung nach einem der vorhergehenden Ansprüche, **dadurch gekennzeichnet, dass** das Aluminiumoxid ein gegebenenfalls hydratisiertes Aluminiumoxid ist.

8. Zusammensetzung nach Anspruch 7, **dadurch gekennzeichnet, dass** es sich bei dem Aluminiumoxid um Böhmit handelt.

9. Zusammensetzung nach einem der vorhergehenden Ansprüche, **dadurch gekennzeichnet, dass** der Mengenanteil der Nanopartikel im Bereich von 0,01 bis 30 Gew.-% und vorzugsweise 0,05 bis 5 Gew.-%, bezogen auf das Gesamtgewicht der Zusammensetzung, liegt.

10. Zusammensetzung nach einem der vorhergehenden Ansprüche, **dadurch gekennzeichnet, dass** das Treibmittel aus Dimethylether alleine oder Gemischen mit Dimethylether besteht.

11. Zusammensetzung nach einem der vorhergehenden Ansprüche, **dadurch gekennzeichnet, dass** das Treibmittel in einer Menge von 2 bis 90 Gew.-% und vorzugsweise 40 bis 80 Gew.-%, bezogen auf das Gesamtgewicht der Zusammensetzung, enthalten ist.

12. Zusammensetzung nach einem der vorhergehenden Ansprüche, **dadurch gekennzeichnet, dass** das kosmetisch akzeptable Medium Wasser und/oder ein kosmetisch akzeptables Lösungsmittel enthält.

13. Zusammensetzung nach Anspruch 12, **dadurch gekennzeichnet, dass** das Lösungsmittel unter den niederen C₁₋₄-Alkooholen, Polyolen, Polyolethern, Aceton und deren Gemischen ausgewählt ist.

14. Zusammensetzung nach Anspruch 13, **dadurch gekennzeichnet, dass** es sich bei dem Lösungsmittel um Ethanol handelt.

15. Zusammensetzung nach Anspruch 12, **dadurch gekennzeichnet, dass** das Wasser weniger als 20 Gew.-% der Zusammensetzung ausmacht.

16. Zusammensetzung nach einem der vorhergehenden Ansprüche, **dadurch gekennzeichnet, dass** die flüssige Phase ferner herkömmliche kosmetische Zusatzstoffe enthält, die unter den adhäsiven Stoffen, Reduktionsmitteln, wie Thiolen, Fettsubstanzen, Verdickungsmitteln, beruhigenden Stoffen, Schaumverhütungsmitteln, Filtern, Antiperspirantien, Ansäuerungsmitteln, Alkalisierungsmitteln, Farbmitteln, Pigmenten, Parfums, Konservierungsmitteln, grenzflächenaktiven Stoffen, fixierenden oder nicht fixierenden Polymeren, flüchtigen oder nichtflüchtigen Siliconen, pflanzlichen Ölen, tierischen Ölen oder Mineralölen, Proteinen, Vitaminen und deren Gemischen ausgewählt sind.

17. Zusammensetzung nach Anspruch 16, **dadurch gekennzeichnet, dass** sie ein Homopolymer oder ein Copolymer von Vinyllactam enthält.

18. Verfahren zur kosmetischen Behandlung der Haare, **dadurch gekennzeichnet, dass** die Zusammensetzung nach einem der vorhergehenden Ansprüche auf die Haare aufgebracht und gegebenenfalls nach einer Einwirkzeit gegebenenfalls gespült wird.

19. Verwendung der aus der Aerosolvorrichtung zerstäubten Zusammensetzung nach einem der Ansprüche 1 bis 14 als Produkt für die Frisurengestaltung, das vorzugsweise nicht ausgespült wird.

## Claims

1. Composition packaged in an aerosol device comprising a liquid phase, which comprises, in a cosmetically acceptable medium, nanoparticles comprising at least 10% by weight of alumina and exhibiting a number-average primary size of between 2 and 200 nm, and a propellant chosen from dimethyl ether, C₃₋₅ alkanes, 1,1-difluoroethane, mixtures of dimethyl ether and of C₃₋₅ alkanes or mixtures of 1,1-difluoroethane and of dimethyl ether and/or of C₃₋₅ alkanes.

2. Composition according to Claim 1, **characterized in that** the number-average primary size of the nanoparticles is between 5 and 50 nm.

3. Composition according to Claim 1 or 2, **characterized in that** the nanoparticles comprising at least 10% by weight of alumina additionally comprise a metal or semimetal oxide other than alumina.

4. Composition according to Claim 3, **characterized in that** the metal or semimetal oxide is chosen from silicon oxide or boron oxide.

5. Composition according to any one of the preceding claims, **characterized in that** the nanoparticles comprise at least 50% by weight of alumina.

6. Composition according to any one of the preceding claims, **characterized in that** the nanoparticles are formed of more than 90% by weight of alumina.

7. Composition according to any one of the preceding claims, **characterized in that** the alumina is an optionally hydrated alumina.

8. Composition according to Claim 7, **characterized in that** the alumina is boehmite.

9. Composition according to any one of the preceding claims, **characterized in that** the amount of the nanoparticles is between 0.01 and 30% by weight, preferably between 0.05 and 5% by weight, with respect to the total weight of the composition.

10. Composition according to any one of the preceding claims, **characterized in that** propellant is composed of dimethyl ether, alone or as a mixture.

11. Composition according to any one of the preceding claims, **characterized in that** the propellant is present in an amount of between 2 and 90% by weight, preferably between 40 and 80% by weight, with respect to the total weight of the composition.

12. Composition according to any one of the preceding claims, **characterized in that** the cosmetically acceptable medium comprises water and/or a cosmetically acceptable solvent.

13. Composition according to Claim 12, **characterized in that** the solvent is chosen from lower C₁-C₄ alcohols, polyols, polyol ethers, acetone, and their mixtures.

14. Composition according to Claim 13, **characterized in that** the solvent is ethanol.

15. Composition according to Claim 12, **characterized in that** the water represents less than 20% by weight of the composition.

16. Composition according to any one of the preceding claims, **characterized in that** the liquid phase additionally comprises conventional cosmetic additives chosen from adhesive agents, reducing agents, such as thiols, fatty substances, thickening agents, softeners, antifoaming agents, screening agents, antiperspirants, acidifying agents, basifying agents, dyes, pigments, fragrances, preservatives, surfactants, fixing or nonfixing polymers, volatile or nonvolatile silicones, vegetable, animal or mineral oils, proteins and vitamins, and their mixtures.

17. Composition according to Claim 16, **characterized in that** it comprises a vinyllactam homopolymer or copolymer.

18. Process for the cosmetic treatment of the hair, **characterized in that** the composition according to any one of the preceding claims is applied to the hair and **in that** rinsing is or is not carried out, after an optional setting time.

19. Use of the composition according to any one of Claims 1 to 17, sprayed from the aerosol device, as styling product, preferably leave-in styling product.
